# EUROPEAN PATENT APPLICATION

(11) **EP 3 269 415 A1**
(43) Date of publication of application: **17.01.2018**
(21) Application number: 17180300.0
(22) Date of filing: 07.07.2017
(51) Int. Cl.: A61M 25/10, A61F 2/958

(54) **MULTI-STAGE BALLOON CATHETER, AND METHOD OF OPERATING SAME IN A CURVED PASSAGEWAY**

(30) Priority: 11.07.2016 US 201662360626 P
(71) Applicant: Cook Medical Technologies LLC, Bloomington, IN 47404 (US)
(72) Inventor: MERK, James, Terre Haute, IN 47802 (US); MAYLE, Brent, Spencer, IN 47460 (US); SPINDLER, Ralf, Solsberry, IN 47459 (US); SKENDER, Davorin, Bloomimgton, IN 47403 (US)
(74) Representative: Garratt, Peter Douglas

(57) **Abstract**

A multi-stage balloon catheter (20) has a deflated state, a first inflation state at a first pressure and a second inflation state at a higher fluid pressure. In the first inflation state, the multi-stage balloon has a plurality of bulb segments (42) separated by waist hoops (40) that allow the multi-stage balloon to conform to match the curvature of a passageway. When pressure is increased in the multi-stage balloon from the first inflation state to the second inflation state, the waist locations expand either by breaking or stretching the waist restraints or by overcoming expansion resistance incorporated into the balloon material at the waist locations. The multi-stage balloon catheter may be used to implant a stent (90) in a manner to conform and match a curved passageway rather than tending to straighten the passageway.

## Description

### Technical Field

The present disclosure relates generally to balloon catheters, such as those used for implanting stents, and more particularly to a multi-stage balloon catheter with enhanced conformability to curved passageways.

### Background

Current balloon devices with one inflation port generally have shown poor conformability when a vessel or passageway is curved. Instead of conforming to the curvature of the vessel, and causing an implanted stent to also match the curvature of the vessel, the balloon tends to drive both the stent and the vessel toward a straight orientation. The stent then is either forced to conform based upon the stiffness of the vessel, or more likely cause the vessel to bend more acutely immediately adjacent one or both ends of the stent.

It is known to shape a balloon to have multiple bulb segments separated by restrained waist segments to produce a multi-stage balloon that tends to conform to a vessel curvature by having adjacent bulb segments pivot about intervening waist segments. For instance, co-owned U.S. Patent Application 2015/0081006 shows a strategy in which suture loops are located at spaced apart locations around a balloon to cause the inflated balloon to have multiple bulb segments separated by constrained waist segments. After initially inflating the balloon to conform to the vessel curvature, a release wire or suture releases the waist segments to expand into the space defined by the vessel wall and the pivoted bulb segments to deploy a stent with a curved confirmation that matches a curvature of the vessel. While this strategy for producing a multi-stage balloon catheter shows promise, there remains room for improvement and reducing costs.

The present disclosure is directed toward one or more of the problems set forth above.

### Summary

In one aspect, a multi-stage balloon catheter includes a catheter that defines an inflation lumen and a centerline. A multi-stage balloon is mounted on the catheter and has an interior fluidly connected to the inflation lumen. A plurality of waist hoops are located at respective waist locations of the multi-stage balloon, and each adjacent pair of waist hoops is separated by a bulb segment of the multi-stage balloon. The multi-stage balloon has a deflated state, a first inflation state and a second inflation state. The first inflation state is characterized by a first fluid pressure in the multi-stage balloon, a hoop tension in the waist hoops holds the waist locations of the multi-stage balloon against expansion, the bulb segments have an expanded diameter greater than a waist diameter, and an adjacent pair of the bulb segments is pivoted with respect to each other about a respective pivot axis that is perpendicular to the centerline and intersects the waist hoop between the pair of adjacent bulb segments. The second inflation state is characterized by a second fluid pressure that is greater than the first fluid pressure, the waist locations are expanded to an enlarged diameter greater than the waist diameter, the bulb segments have at least the expanded diameter, and the adjacent pair of bulb segments remain pivoted with respect to each other about the respective pivot axes.

In another aspect, a method of operating a multi-stage balloon catheter includes positioning the multi-stage balloon in a curved passageway with the multi-stage balloon in the deflated state. The multi-stage balloon is then inflated to a first inflation state with fluid at a first fluid pressure. The waist locations of the multi-stage balloon are held against expansion with hoop tension in the waist hoops while in the first inflation state. The centerline of the catheter conforms to match the curved passageway responsive to an interaction of the bulb segments with a wall that defines the curved passageway. The interaction pivots adjacent bulb segments relative to each other about a respective pivot axis that is perpendicular to the centerline and intersects the waist hoop that separates the adjacent bulb segments. The waist locations are then expanded into a space defined by the wall and the multi-stage balloon by changing the multi-stage balloon from the first inflation state to the second inflation state by increasing the fluid pressure from the first fluid pressure to the second fluid pressure, while the centerline remains conformed to match the curved passageway.

### Brief Description of the Drawings

Fig. 1 shows a balloon catheter with a non-compliant balloon in an expanded state;
Fig. 2 is the balloon catheter of Fig. 1 with the balloon in a deflated state;
Fig. 3 is a side view of the balloon catheter of Fig. 2 after being changed into a multi-stage balloon catheter according to the present disclosure by the addition of waist restraints at selected waist locations along the balloon catheter centerline;
Fig. 4 is a side view of the multi-stage balloon catheter of Fig. 3 in a first inflation state and curved to match a curved passageway;
Fig. 5 is a side view of the multi-stage balloon catheter of Fig. 4 in a second inflation state;
Fig. 6 shows a multi-stage balloon catheter in a deflated state carrying a stent within a curved passageway;
Fig. 7 is a side schematic view of the multi-stage balloon catheter of Fig. 6 after being inflated to a first inflation state;
Fig. 8 is a schematic side view of the multi-stage balloon catheter of Figs. 6 and 7 in a second inflation state;
Fig. 9 is a schematic view of another multi-stage balloon catheter according to the present disclosure in a deflated state carrying a stent in a curved passageway;
Fig. 10 is a schematic side view of the multi-stage balloon catheter and stent of Fig. 9 in a first inflation state;
Fig. 11 is a schematic side view of the multi-stage balloon catheter of Figs. 9 and 10 in a second inflation state;
Fig. 12 is a side schematic view of an multi-stage balloon according to the present disclosure covered by a breakable mesh of different densities at the respective waist and bulb segments of the multi-stage balloon;
Fig. 13 is a side schematic view of a multi-stage balloon similar to Fig. 12 except with the mesh being thicker filaments at the waist segments relative to the filament mesh at the bulb segments;
Fig. 14 is a schematic side view similar to Figs. 12 and 13 except having no breakable mesh between the waist hoops, which are defined by breakable mesh hoops;
Fig. 15 is a schematic side view of a film version of a multi-stage balloon catheter in a deflated state carrying a stent in a curved passageway;
Fig. 16 is a schematic side view of the multi-stage balloon catheter of Fig. 15 in a first inflation state;
Fig. 17 is a schematic view of the multi-stage balloon catheter of Figs. 15 and 16 in a second inflation state;
Fig. 18 is a schematic side view of a multi-stage balloon catheter in which the waist and bulb segments of the balloon are covered by brittle and ductal film, respectively;
Fig. 19 is a schematic side view of a film covered version of a multi-stage balloon catheter similar to Fig. 18, except the waist segments have a breakable thicker film relative to a thin film covering the bulb segments;
Fig. 20 is a schematic side view of still another film version of a multi-stage balloon catheter in which the waist segments have un-weakened film and the bulb segments have perforated film to facilitate breakage at the second and first fluid pressures, respectively;
Fig. 21 is a schematic side view of still another film version in which the bulb segments are not covered by film but the waist segments have breakable film hoops that break when the fluid pressure is increased from the first fluid pressure to the second fluid pressure;
Fig. 22 shows still another multi-stage balloon catheter in a deflated state;
Fig. 23 is a side schematic view of the multi-stage balloon catheter of Fig. 22 after being inflated to a first inflation state in a curved passageway; and
Fig. 24 is a schematic side view the multi-stage balloon catheter of Figs. 22 and 23 in a second inflation state.

### Detailed Description

A multi-stage balloon catheter according to the present disclosure can take a wide variety of forms and be constructed from various materials. In all cases, the balloon of the multi-stage balloon catheter will have the ability to change from a deflated state, to a first inflation state and then a second inflation state. In the first inflation state, the multi-stage balloon will include a plurality of bulb segments separated by smaller diameter waist hoops. At a minimum, the multistage balloon would include a plurality of waist hoops that each separate a pair of bulb segments. The second inflation state is characterized by a second fluid pressure that is greater than the first fluid pressure, and the waist locations are expanded to an enlarged diameter. This strategy allows the multi-stage balloon catheter to conform to match a curved passageway and then more fully expand in the curved passageway. Balloons for the multi-stage balloon catheter according to the present disclosure can be made from compliant balloon materials, non-compliant balloon materials, semi-compliant or a hybrid combination. Waist hoops according to the present disclosure can be incorporated into the balloon material, be made from a second material mounted around but unattached to the underlying balloon, be attached to an outer surface of the balloon, or some combination of these structural strategies. Waist hoops according to the present disclosure can be comprised of a single filament, multiple filaments, a mesh, a film or even a difference in balloon wall thickness without departing from the intended scope of the present disclosure. Among other uses, multi-stage balloon catheters according to the present disclosure can find potential use in delivery of plastically expanded stents, especially in curved passageways. Multistage balloon catheters according to this disclosure may also be used for post dilation of self expanding stents, for angioplasty or other potential uses known in the art.

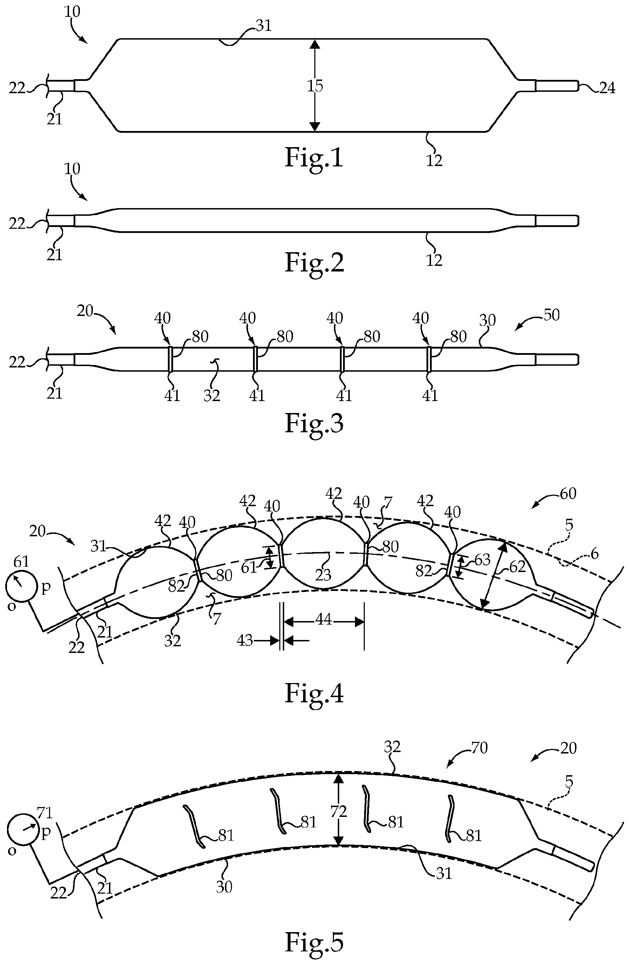

Referring initially to Figs. 1-5, one strategy for making a multi-stage balloon catheter according to the present disclosure may begin with a conventional balloon catheter 10 that includes a non-compliant balloon 12 mounted on a catheter 21 near an end tip 24. Catheter 21 will typically include an inflation lumen 22 that opens to the interior 31 of balloon 12; and may include a separate wire guide lumen that is not shown. Non-compliant balloon 12 may have a uniform diameter 15. Non-compliant balloon materials include, but are not limited to a nylon family of materials, polyethylene terephthalate (PET) and other similarly behaving materials known in the art. Fig. 2 shows balloon catheter 10 in its deflated state 50, which is typically associated with a configuration when the balloon catheter is being maneuvered to a treatment site. The balloon catheter 10 of Figs. 1 and 2 may be made into a multi-stage balloon catheter 20 according to the present disclosure by including a plurality of waist hoops 40 at spaced apart waist locations 41 on the outer surface 32 of the now multi-stage balloon 30. In this embodiment, the waist hoops 40 may take the form of waist restraints 80 formed of a breakable filament hoop 82. The breakable filament hoops 82 are designed to break at a hoop tension corresponding to when the multi-stage balloon 30 is inflated above a predetermined fluid pressure. Although not shown, the breakable filament hoops 82 may be connected to one another by another filament or suture, which may extend all the way to the proximal end of multi-stage balloon catheter 20 so that the filament hoops 82 can be easily retrieved after being broken. On the other hand, the breakable filament hoops 82 may be attached to the outer surface 32 of the balloon 30, as shown.

Each of the waist hoops 40 is separated by a bulb segment 42 of the multi-stage balloon 30. In the illustrated embodiment, each of the waist hoops 40 has a width 43 along a centerline 23 that is less than a distance 44 along the centerline 23 between adjacent waist hoops 40. However, those skilled in the art will appreciate that multi-stage balloons with waist hoops of varying width, with waist hoop widths greater than a width of one or more of the bulb segments, with varying width bulb segments, or some combination of these features would also fall within the intended scope of this disclosure. Furthermore, bulb segments of different intermediate and/or final diameters would also fall within the intended scope of this disclosure. Fig. 3 shows a multi-stage balloon in a deflated state 50, Fig. 4 shows the balloon in a first inflation state 60, and Fig. 5 shows the balloon 30 in its second inflation state 70. First inflation state 60 corresponds to a first fluid pressure 61, and the second inflation state 70 corresponds to a second fluid pressure 71 that is greater than the first fluid pressure 61, and sufficient to cause the waist hoops to have a hoop tension sufficient to break the breakable filament hoops 82. In other words, the breakable filament hoops 82 become broken waist restraints 81 when the fluid pressure is increased from the first fluid pressure 61 to the second fluid pressure 71. In this embodiment, the breakable filament hoops 82 are shown attached to the outer surface 32 of the multi-stage balloon 30 so that the broken waist restraints 81 stay with the multi-stage balloon catheter 20 after a treatment is completed and the multi-stage balloon catheter 20 is withdrawn from the curved passageway 5.

The first inflation state 60 is characterized by the first fluid pressure 61 in the multi-stage balloon 30, and a hoop tension in the waist hoops 40 that holds the waist locations 41 of the multi-stage balloon 30 against expansion. The bulb segments 42 have an expanded diameter 62 that is greater than a waist diameter 63. Each adjacent pair of the bulb segments 42 maybe pivoted with respect to each other about a pivot axis that is perpendicular to the centerline 23 and intersects the waist hoop 40 between the pair of adjacent bulb segments 42. Each respective pivot axis is not visible in Figs. 4 and 5 as the pivot axes extend into and out of the drawing sheet through the respective waist hoops 40. Those skilled in the art will appreciate that the pivot axes may, and likely will be, angled with respect to each other to reflect a three dimensional shape of a curved passageway.

The second inflation state 70 is characterized by a second fluid pressure 71 that is greater than the first fluid pressure 61, and the waist locations are expanded to an enlarged diameter 72 that is greater than the waist diameter 63. The bulb segments 42 have at least the expanded diameter 62, and the adjacent pairs of bulb segments 42 remain pivoted with respect to each other about the respective pivot axes extending in and out of the page through the waist hoops 40. The second inflation state 70 (Fig. 5) may include balloon 30 having creases toward the inner radius where less balloon surface area is needed to fill curved passageway 5. Figs. 4 and 5 show the multi-stage balloon catheter as it might appear in a curved passageway 5 in which an interaction between a wall 6 that defines the curved passageway 5 interacts with the bulb segments 42 to cause the pivoting action about the pivot axes extending through the respective waist hoops 40. When the multi-stage balloon catheter 20 is changed from the first inflation state 60 of Fig. 4 to the second inflation state 70 of Fig. 5, the breakage of the waist restraints 80 allows the balloon 30 to at least partially fill the space 7 previously defined by the wall passage 6 and the exterior surface 32 of the multi-stage balloon 30.

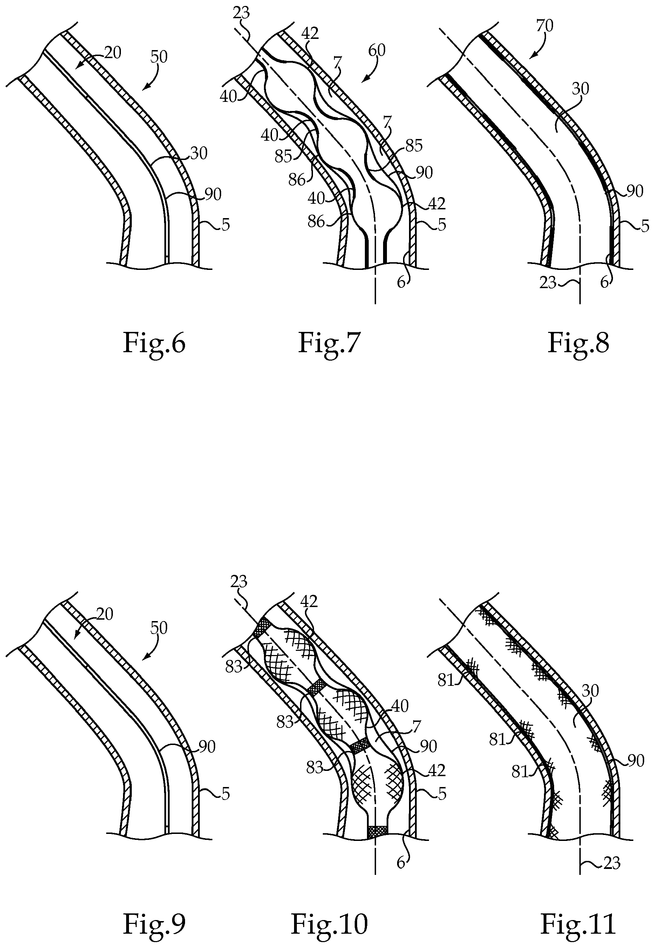

Referring now to Figs. 6, 7 and 8, a multi-stage balloon catheter 20 includes waist hoops 40 that are incorporated as part of the material for the multi-stage balloon 30, as opposed to being separate breakable waist constraints 80 as in the embodiment shown in Figs. 1-5. Although different embodiments are shown and described, identical numbers are used to refer to identically named features throughout this disclosure. The multi-stage balloon catheter 20 is shown in its deflated state 50 (Fig. 6), in its first inflation state 60 (Fig. 7) and its second inflation state 70 (Fig. 8). This embodiment also differs from the earlier embodiment in that multi-stage balloon catheter 20 includes a plastically expanded stent 90 mounted on the multi-stage balloon 30 for implantation in the curved passageway 5. In this embodiment, multi-stage balloon 30 may have different compliance characteristics at the bulb segments 42 relative to the waist hoops 40. For instance, the bulb segments may include relatively stiff elastomers like urethane, neoprene, silicon, nylon, PET or other thermoplastic elastomers that are relatively non-compliant. The "relative" term is used here in comparing waist hoops to bulb segments only. The waist hoops 40 may be manufactured from semi-compliant or compliant material in the balloon wall 85 at the waist hoops 40. This variable compliance may be achieved through the use of plasticizers to adjust the compliance at the waist hoops 40 through plasticizer exposure via time, temperature, concentration, type and/or composition. In general, longer plasticizer exposure provides increased flexibility, durability and increases compliance. Alternatively, the multi-stage balloon 30 may be formed of a compliant or semi-compliant material, but the waist hoops are achieved through wall thickness control. For instance, the wall thickness 86 of the multi-stage balloon 30 could be thinner in the bulb segments 42, and wall thickness 85 may be thicker at the waist hoops 40 so that the multi-stage balloon 30 assumes the shape shown in Fig. 7 when in its first inflation state 60 at a first or intermediate fluid pressure. The shape of the multi-stage balloon 30 will change from the first inflation state 60 of Fig. 7 to the second inflation state 70 by raising the fluid pressure within the multi-stage balloon 30. By having the intermediate state shown in Fig. 7, the multi-stage balloon catheter and the carried stent 90 can conform to match to the curved passageway 5 via the interaction of the wall 6 with the bulb segments 42 to cause the centerline 23 of the multi-stage balloon catheter to match the curve passageway 5. When the stent 90 is fully expanded by full inflation of the multi-stage balloon 30 in Fig. 8, the stent 90 may be fully expanded but expanded in a way that matches the curved passageway 5 instead of tending to straighten out the passageway as in some prior art devices. The embodiment of Figs. 6-8 is also of interest for showing waist hoops 40 that may be achieved by having a hybrid balloon structure that differs at the waist hoops 40 from the bulb segments 42 either by varying compliance materials or by differing wall thicknesses of the balloon material, or a combination of both. Furthermore, the multi-stage balloon 30 of Figs. 6-8 could be manufactured with a composite balloon materials that differ at waist hoops 40 from the bulb segments 42 in order to achieve the shapes shown in Figs. 7 and 8. Thus, the embodiment shown in Figs. 6-8 differs from the embodiment shown in Figs. 1-5 by the waist hoops 40 having greater hoop elasticity than the bulb segments 42 beyond the first inflation state 60. The waist hoops enlarge responsive to an increase from the first fluid pressure to the second fluid pressure associated with the second inflation state 70 shown in Fig. 8. Balloon 30 may include an appropriate feature, such as a restraint, to keep the bulb segments 42 from over expanding when the inflation pressure is increased to the second fluid pressure 71.

Referring now to Figs. 9-11. A multi-stage balloon catheter 20 is similar to the embodiment of Figs. 1-5, except breakable mesh hoops 83 are used instead of the breakable filament hoop 82 associated with the earlier embodiment. Like the earlier embodiments, the multi-stage balloon catheter 20 is shown in its deflated state 50, its first inflation state 60 and second inflation state 70 in Figs. 9, 10 and 11, respectively. In this embodiment, the waist hoops 40 are made up of breakable mesh hoops 83. As with the earlier embodiment, the interaction between the bulb segments 42 with the wall 6 that defines a curve passageway 5 causes the centerline 23 of the multi-stage balloon catheter 20 to conform to match that of curve passageway 5. The breakable mesh hoops 83 could be designed to break at predetermined locations on the mesh when the multi-stage balloon 30 is inflated with increased pressure when transitioning from the first inflation state 60 to the second inflation state 70. For example, the breakable mesh filaments could have included thinner mesh filaments that would break at a pre-determined hoop tension associated with a pre-determined inflation pressure, or all filaments could be designed to break at a pre-determined inflation pressure when the hoop tension exceeded some threshold associated with the increased fluid pressure of the second inflation state 70. In one variation, the breakable mesh hoops 83 could be formed from a bioresorbable material, and/or the breakable mesh hoops 83 could be attached to the outer surface of the balloon even after being broken to be withdrawn with the multi-stage balloon catheter 20 after a treatment procedure is completed.

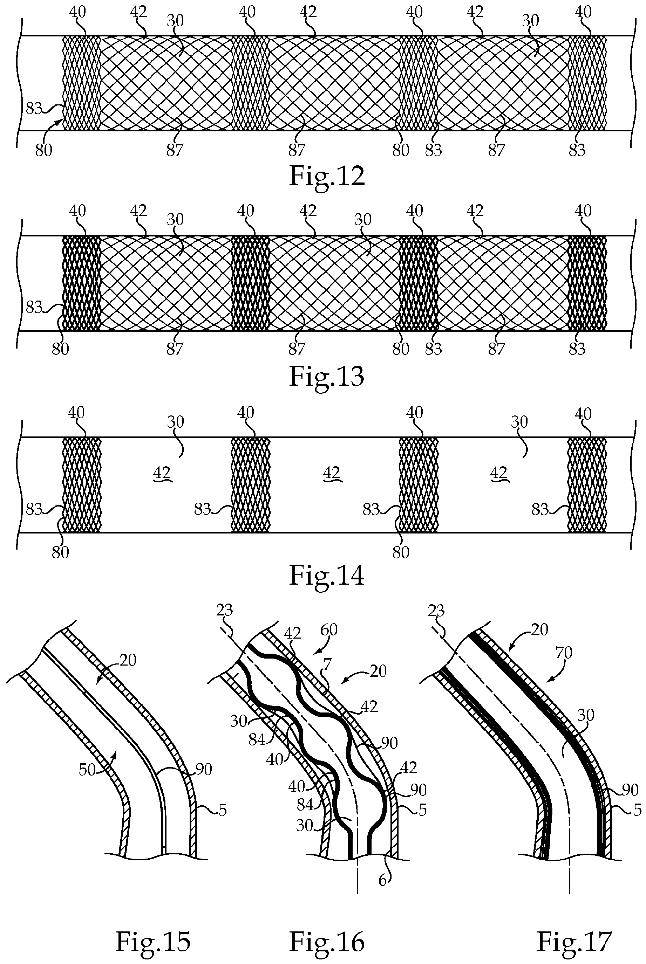

Referring now in addition to Figs. 12-14, several different alternative breakable mesh hoop embodiments are illustrated. For instance, the alternatives shown in Figs. 12 and 13 show examples where a mesh covers the entire multi-stage balloon 30 but either the cross sectional area or density of the filaments that make up of the breakable mesh hoop 83 are stronger than the portions of the mesh 87 that cover the bulb segments 42. In these examples, the portions of the mesh 87 covering the bulb segments could either stretch to accommodate expansion of the bulb segments 42, or they themselves could break responsive to multi-stage balloon being inflated from the deflated state 50 to the first inflation state 60 as shown in Fig. 10. In either case, these strategies of covering the entire or a majority of the length of the multi-stage balloon 30 with a varying breakable mesh could be chosen as a alternative way of constructing the multi-stage balloon catheter 20. The present disclosure also contemplates a version as shown in Fig. 14 in which the breakable mesh only appears at the waist restraints 80 for the waist hoops 40, leaving no mesh in the areas of the bulb segments 42. The present disclosure also contemplates constructing the mesh at least partially from bioresorbable materials such as polylactic acid (PLA), polyglycolic acid (PGA) or various combinations of these two materials and other materials. Additionally, any material that would not be bioresorbable could be composed of filaments similar to that used for sutures whether permanent or resorbable. In addition, the mesh material may be adhered to the outer surface of the multi-stage balloon 30 through an appropriate strategy, such as sonic welding, or possibly be bonded in a same area where the balloon 30 is bonded to the underlying catheter 21. In this way, the broken mesh material would remain with the balloon upon withdrawal from the curved passageway 5.

The breakable mesh hoops 83 could also be formed of more brittle materials such as polylactic acid, polylactide-co-glycolide, polycaprolactone, polydioxanone, and maybe polyamino acids including leucine, lysine and glutamate. Instead of the bioresorbable materials mentioned above, the breakable mesh hoops could be also made from polyester textiles formed as an ultra-thin fabric-textile with interstitial space depending on weaving dimensions. In such a case, a textile would also be considered a mesh in the context of the present disclosure. In still another case, a brittle alternative might be used to construct a mesh from polyester sutures that are small enough in cross section and by controlling the number of filaments to offer a parametric control over failure. Stretchable mesh materials may include polyethylene sutures that exhibit ductility when put under tension. Alternatively, PTFE/ePTFE could be castable into thin films and remain flexible and can be thermoformed into whatever shape desired. Thin strands of material could either break due to small cross section or ductile/stretching with thicker filaments. Furthermore, certain filaments used in either the breakable mesh hoops 83 or the breakable filament hoop 82 can also be mechanically modified by pulling filaments until necking occurs to create thinner areas where the fracture will occur when inflating to the second inflation state 70. Furthermore, breaking locations can be created by indentations or scoring to further control the location of where a fracture might occur.

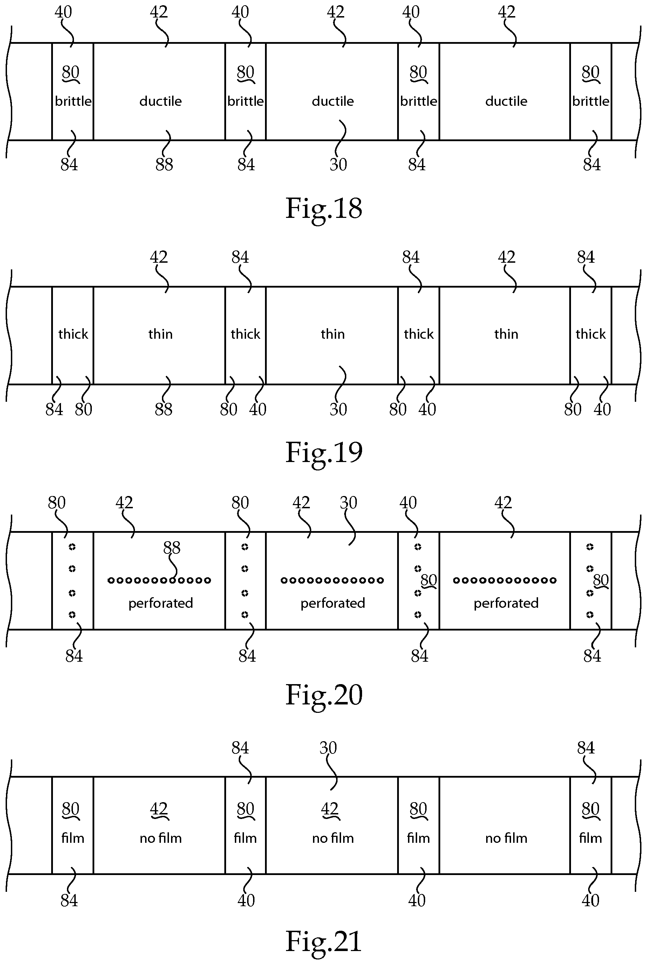

Referring now to Figs. 15-17, still another embodiment of a multi-stage balloon catheter 20 is shown in its deflated state 50, a first inflation state 60 and a second inflation state 70, respectively. This embodiment is similar to the previous embodiment except that instead of a breakable mesh hoops 82, some or all of the balloon is covered by a breakable film. For instance, multi-stage balloon 30 could include breakable film hoops 84 that are mounted on or attached to the outer surface of the multi-stage balloon 30, and designed to break when the hoop tension in the breakable film hoop 84 exceeds a predetermined tension associated with increasing the balloon inflation pressure from the first inflation state 60 to the second inflation state 70. Figs. 18-20 show several different strategies for utilizing breakable film hoops 84 in a multi-stage balloon catheter 20 according to the present disclosure. For instance, as shown in Fig. 18, the entire balloon could be covered in film but the film 88 covering the bulb segments 42 could be manufactured to be ductal whereas the film at the waist hoops 40 could be brittle so as to break at a predetermined tension. Or, the film could have variable ductility to stretch at different pressures but without breaking. Fig. 19 shows an alternative version in which the breakable film hoop 84 could be thicker and act as a waist constraints 80 whereas the film covering the bulb segments 42 could be thinner, and thus the thin film would break when changing the balloon from the deflated state 50 to the first inflation state 60, but the breakable film hoops 84 would not break until increasing fluid pressure from the first inflation state 60 to the second inflation state 70. Or, the film would have thickness differences to stretch at different pressures, but without breaking. Fig. 20 shows still another alternative in which a somewhat uniform film covers the entire multi-stage balloon 30 but the balloon is perforated or scored or otherwise intentionally weakened where it covers the bulb segments 42 but the unperforated breakable film hoops 84 need to be designed to act as waist restraints 80 until the fluid pressure increases toward the second inflation state 70. An alternative version might include perforations in waist hoops 40 (dashed lines) to engineer breakage of the waist hoops 40 at a prescribed fluid pressure. Fig. 21 shows that still another alternative where breakable film hoops exist at the waist hoops 40 and no film is included covering the bulb segments 42. Or, the film at the waist hoop could be engineered to hold at the first pressure, but stretch without breaking at the higher pressure. The film according to the present disclosure could be applied to the balloon surface, or could be applied after the balloon is placed in its deflated state 50 so that the film only contacts exposed portions of the folded balloon in its deflated state 50. The film material could be attached to the outer surface of the balloon 30 so that even when broken, the film would stay with the balloon and be removed from the curved passageway 5 with the multi-stage balloon catheter 20 after a treatment has been performed, such as implanting a stent 90 in curve passageway 5.

Because breakage of the films contemplated for the present disclosure could release smaller particles, the films could be made from bioresorbable materials. These materials include but are not limited to PLA, PGA, PCL, PDX and polyaminoacids. Furthermore, polyester can be used as an ultra-thin film in the form of a fabric or textile, which would also be considered a film or mesh according to the present disclosure. Parylene may also be castable as a film and may be brittle or ductile depending upon formulation. Other stretchable or ductile film formulations may include PTFE or high molecular weight polyethylene. Failure of the breakable film hoops 84 may be achieved through perforations, through the thickness of the films, by making the film more brittle by utilizing a random failure analysis, and maybe even mechanical modification through indenting or scoring the film with mechanical tools to created a break location.

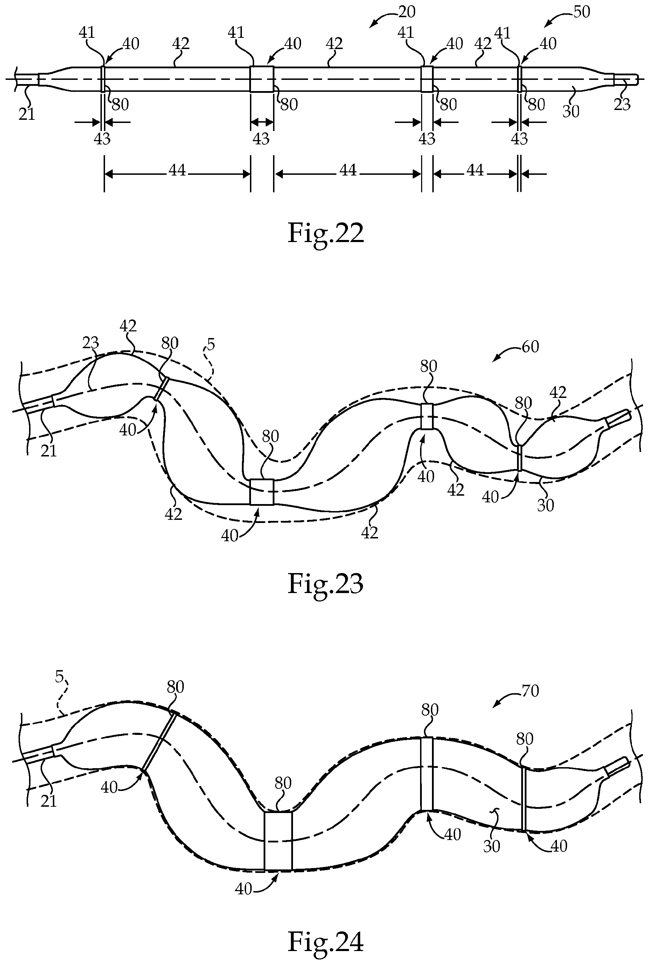

Referring now to Figs. 22-24, still another multi-stage balloon catheter 20 according to the present disclosure is illustrated in its respected deflated state 50, first inflation state 60 and second inflation state 70. Like the earlier embodiments, this multi-stage balloon catheter 20 includes a multi-stage balloon 30 mounted about a catheter 21. This embodiment differs in that the four waist hoops 40 are at variable waist locations 41 along centerline 23, and three of the waist hoops 40 have different widths 43. This embodiment also is different in that the distances 44 between adjacent waist hoops 40 are also different from each other. Thus, the present disclosure contemplates waist hoops 40 of various widths 43 and separated by variable distances 44. In this embodiment, the waist hoops are defined by expandable waist restraints 80 that are attached to multi-stage balloon 34 formed as different thickness or material from that of the portions of multi-stage balloon 30 that make up bulb segments 42. Those skilled in the art will appreciate that the variability illustrated by the embodiment of Figs. 22-24 permits device engineers greater flexibility in designing specific flexibility characteristics to suit a particular procedure and anatomy. Furthermore, those skilled in the art will appreciate that differing widths 43 for the waist hoops 40 may result in different bending characteristics about those respective hoops. Furthermore, the present disclosure contemplates waist hoops 40 of different diameters in the first inflation state 60 to further allow for varying flexibility and pivoting characteristics of the flanking bulb segments 42. For instance, one could expect greater flexibility about waist hoops 40 having a smaller diameter in the first inflation state 60 than counterpart waist hoops 40 having a larger diameter in the first inflation state 60.

### Industrial Applicability

The present disclosure finds general applicability with balloon catheters and any of their assorted uses known in the art. The present disclosure finds specific applicability for balloon catheters for use in curved passageways. Finally, the present disclosure finds specific applicability for being used for implanting a stent in a curved passageway in a way that conforms to the curvature of the curved passageway, rather than tending to straighten the curved passageway as in the prior art.

Referring now to Figs. 3-5, 6-8, 9-11, 15-17, and 22-24 a method of operating a multi-stage balloon catheter 20 includes positioning the multi-stage balloon 30 in a curved passageway with the multi-stage balloon 30 in a deflated state 50. The multi-stage balloon 30 is then inflated to a first inflation state 60 with fluid at a first fluid pressure. Waist locations 41 of the multi-stage balloon 30 are held against expansion with hoop tension in waist hoops 40 while in the first inflation state 60. A centerline 23 of the multi-stage balloon catheter 20 conforms to match the curved passageway 5 responsive to an interaction of the bulb segments 42 with a wall 6 that defines the curved passageway 5. The interaction causes the adjacent bulb segments 42 to pivot relative to each other about a pivot axis that is perpendicular to the centerline 23 and intersects the respective waist hoops 40 that separate adjacent pairs of the bulb segments 42. Thereafter, the waist locations 41 of the multi-stage balloon 30 are expanded into a space 7 defined by the wall 6 and the multi-stage balloon 30 by changing the multi-stage balloon from the first inflation state 60 to a second inflation state 70 by increasing fluid pressure from the first fluid pressure to a second fluid pressure, while the centerline 23 remains conformed to match the curved passageway 5.

In some embodiments, the waist hoops 40 include waist restraints 80 that are mounted about the multi-stage balloon 30 at each of the waist locations 41. The waist restraints 80 break or stretch without breaking responsive to a fluid pressure increase from the first fluid pressure to the second fluid pressure. In some embodiments, the waist restraints 80 may be manufactured from a bioresorbable material such that the step of breaking the waist restraints 80 includes detaching bioresorbable material of the waist restraint 80 from the multi-stage balloon catheter 20. In other embodiments, the waist hoops 40 are incorporated as part of the balloon material such that the waist hoops have greater elasticity that the bulb segments 42 beyond the first inflation state 60. This strategy, for instance, might be accomplished by making the bulb segments 42 from non-compliant balloon material or by having some other external constraint that prevents overexpansion of the bulb segments 42. The waist hoops 40 then enlarge responsive to an increase from the first fluid pressure 61 to the second fluid pressure 71. In the embodiment of Figs. 3-5, the multi-stage balloon 30 may have a uniform diameter 15 at the waist locations 41 and the bulb segments 42. In some specific applications, a stent 90 is expanded responsive to changing a multi-stage balloon from the deflated state 50 to the first inflation state 60 and then on to the second inflation state 70. This strategy may include conforming the stent 90 to match the curved passageway 5 responsive to changing the multi-stage balloon from the deflated state 50 to the second inflated state 70.

The disclosed or other methods according to the invention may usefully be performed ex vivo in an appropriate structure that defines a curved passageway. Such a structure may be transparent or otherwise provide visbility of the method as executed. An ex vivo method may be useful for the purposes - for example - of demonstration, training or testing.

It should be understood that the above description is intended for illustrative purposes only, and is not intended to limit the scope of the present disclosure in any way. Thus, those skilled in the art will appreciate that other aspects of the disclosure can be obtained from a study of the drawings, the disclosure and the appended claims.

## Claims

1. A multistage balloon catheter comprising:
a catheter that defines an inflation lumen and a centerline;
a multistage balloon mounted on the catheter and having an interior fluidly connected to the inflation lumen;
a plurality of waist hoops at respective waist locations of the multistage balloon, and each adjacent pair of waist hoops being separated by a bulb segment of the multistage balloon;
the multistage balloon having a deflated state, a first inflation state and a second inflation state;
the first inflation state being **characterized by** a first fluid pressure in the multistage balloon, hoop tension in the waist hoops holding the waist locations of the multistage balloon against expansion, the bulb segments having an expanded diameter greater than a waist diameter, and each adjacent pair of the bulb segments being pivoted or pivotal with respect to each other about a pivot axis that is perpendicular to the centerline and intersects the waist hoop between the pair of adjacent bulb segments; and
the second inflation state being **characterized by** a second fluid pressure that is greater than the first fluid pressure, the waist locations are expanded to an enlarged diameter greater than the waist diameter, the bulb segments have at least the expanded diameter, and the adjacent pair of bulb segments remain pivoted with respect to each other about the respective pivot axis.

2. The multistage balloon catheter of claim 1 wherein each of the waist hoops is configured to yield circumferentially in response to increasing of fluid pressure in the multistage balloon beyond said first fluid pressure toward said second fluid pressure.

3. The multistage balloon catheter of claim 1 or claim 2 wherein each of the waist hoops includes a waist restraint mounted about the multistage balloon at each of the waist locations;
each of the waist restraints breaks responsive to a fluid pressure increase from the first fluid pressure to the second fluid pressure, preferably wherein the waist restraint includes at least one breakable filament hoop mounted about an outer surface of the multistage balloon; a mesh hoop mounted about an outer surface of the multistage balloon; a film hoop mounted about an outer surface of the multistage balloon; or a waist restraint formed of a bioresorbable material.

4. The multistage balloon catheter of any preceding claim wherein the multistage balloon is formed of a noncompliant material and/or with a uniform diameter at the waist locations and the bulb segments.

5. The multistage balloon catheter of any one of claims 1 to 3 wherein the multistage balloon is formed of a compliant material with a wall thickness at the waist locations that is greater than a wall thickness at a center of the bulb segments or with a hoop compliance at the waist locations that is less than a hoop compliance at a center of the bulb segments.

6. The multistage balloon catheter of any preceding claim wherein the waist hoops have a greater hoop elasticity than the bulb segments in the first inflation state; and
the waist hoops enlarge responsive to an increase from the first fluid pressure to the second fluid pressure.

7. The multistage balloon catheter of any preceding claim wherein each of the waist hoops has a width along the centerline that is less than a distance along the centerline between adjacent waist hoops.

8. The multistage balloon catheter of any preceding claim including a stent mounted on the multistage balloon in the deflated state.

9. A method of operating ex vivo a multistage balloon catheter that includes a catheter that defines an inflation lumen and a centerline; a multistage balloon mounted on the catheter and having an interior fluidly connected to the inflation lumen; a plurality of waist hoops at respective waist locations of the multistage balloon, and each adjacent pair of waist hoops being separated by a bulb segment of the multistage balloon; the multistage balloon having a deflated state, a first inflation state and a second inflation state, and the method comprising the steps of:
positioning the multistage balloon in a curved passageway with the multistage balloon in the deflated state;
inflating the multistage balloon to the first inflation state with fluid at a first fluid pressure;
holding the waist locations of the multistage balloon against expansion with hoop tension in the waist hoops while in the first inflation state;
conforming the centerline to match the curved passageway responsive to an interaction of the bulb segments with a wall that defines the curved passageway, and wherein the interaction pivoting adjacent bulb segments relative to each other about a respective pivot axis that is perpendicular to the centerline and intersects the waist hoop that separates the adjacent bulb segments; and
expanding the waist locations into space defined by the wall and the multistage balloon by changing the multistage balloon from the first inflation state to the second inflation state by increasing fluid pressure from the first fluid pressure to a second fluid pressure while the centerline remains conformed to match the curved passageway.

10. The method of claim 9 wherein each of the waist hoops includes a waist restraint mounted about the multistage balloon at each of the waist locations; and
breaking each of the waist restraints responsive to a fluid pressure increase from the first fluid pressure to the second fluid pressure; preferably wherein the breaking step includes detaching bioresorbable material of the waist restraint from the multistage balloon catheter.

11. The method of any of claims 9-10 wherein
the waist hoops enlarge responsive to an increase from the first fluid pressure to the second fluid pressure.

12. The method of any of claims 9-11 wherein the multistage balloon has a uniform diameter at the waist locations and the bulb segments.

13. The method of any of claims 9-12 including constraining the bulb segments from further expansion when increasing fluid pressure from the first fluid pressure toward the second fluid pressure; preferably by forming at least the bulb segments of the multistage balloon from a non-compliant material.

14. The method of any of claims 9-13 including expanding a stent responsive to changing the multistage balloon from the deflated state to the second inflation state.

15. The method of any of claims 9-14 including conforming a stent to match the curved passageway responsive to changing the multistage balloon from the deflated state to the second inflation state.
